# NEUE EUROPÄISCHE PATENTSCHRIFT

(11) **EP 0 872 217 B2**
(45) Veröffentlichungstag und Bekanntmachung des Hinweises auf die Entscheidung über den Einspruch: **27.12.2006**
(45) Hinweis auf die Patenterteilung: 02.04.2003
(21) Anmeldenummer: 98106881.0
(22) Anmeldetag: 16.04.1998
(51) Int. Cl.: A61C 1/07, A61C 1/05, A61B 17/16

(54) **Medizinisches oder dentalmedizinisches Behandlungsinstrument zur spanabhebenden Bearbeitung von Körpergewebe oder einem Ersatzstoff mit einem abrasiven Werkzeug**
Medical or dental instrument for cutting body tissue or a replacement material with an abrasive tool
Instrument médical ou dentaire d'usinage par abrasion de tissus corporels ou d'un matériau de remplacement

(30) Priorität: 18.04.1997 DE 19716416; 13.10.1997 DE 19745245; 21.11.1997 DE 19751584
(43) Veröffentlichungstag der Anmeldung: 21.10.1998
(60) Teilanmeldung: 03001043.3
(73) Patentinhaber: KALTENBACH & VOIGT GMBH & CO, 88400 Biberach/Riss (DE)
(72) Erfinder: Gugel, Bernd, 89079 Ulm (DE); Mössle, Walter, 88441 Mittelbiberach (DE); Löhne, Gerd, 88400 Biberach-Rissegg (DE); Xeller, Uli, 88400 Biberach (DE)
(74) Vertreter: Schmidt-Evers, Jürgen

(56) Entgegenhaltungen:
- DE-A1- 2 730 137
- FR-A- 2 132 434
- FR-A- 2 505 172
- FR-A- 2 550 439
- FR-A- 2 613 090
- US-A- 4 484 893
- Dental Products Report Europe, Sept.1996, S.43
- Beschreibung Zahnsteinentferner AIRSON
- Konstruktionszeichnungen 1/2-2/2 vom 07.12.95
- Rechnung über zwei Detartaratore AIRSON vom 26.11.96

## Beschreibung

Die Erfindung bezieht sich auf ein medizinisches oder dentalmedizinisches Behandlungsinstrument nach dem Oberbegriff des Anspruchs 1.

Zur spanabhebenden Bearbeitung von Körpergewebe oder einem Ersatzstoff (Gewebeersatz oder einer Prothese) sind Behandlungsinstrumente mit einem abrasiven Werkzeug und einem Schwingungsantrieb einer vorzugsweise im Schall- oder Ultraschallbereich liegenden Frequenz bekannt. Ein solches Behandlungsinstrument ist z.B. in der WO 96/14024 beschrieben. Es weist ein längliches Handstück in Form eines hülsenförmigen Gehäuses auf, in dessen vorderem Bereich ein Schwingteil elastisch nachgiebig gelagert ist, das im Funktionsbetrieb durch einen Schwingungserreger in Schwingungen versetzbar ist und in seinem vorderen Bereich durch eine Haltevorrichtung mit dem Werkzeug lösbar verbindbar ist. Im hinteren Endbereich weist das Handstück ein Kupplungsteil auf, insbesondere ein Schraub- oder Steckkupplungsteil, mit dem es mit einem sogenannten Anschlußstück einer Versorgungseinrichtung in Form eines flexiblen Schlauches kuppelbar ist, der sich zu einem Versorgungs- und Steuergerät erstreckt und Medienleitungen zur Energieversorgung und Zuführung von Behandlungsmedien wie z.B. Wasser, Luft oder ein Spray, enthält.

Ein Behandlungsinstrument dieser Art läßt sich im dentalmedizinischen Bereich auch als Zahnsteinentfemungsgerät verwenden, wobei ein solches, aus der österreichischen Patentschrift 379 505 bekanntes Behandlungsinstrument ein Werkzeug mit einer keilförmigen Arbeitsspitze aufweist.

In der FR 2 613 090 A ist ein Behandlungsinstrument der eingangs angegebenen Art beschrieben. Dieses vorbekannte Behändlungsinstrument weist eine manuell bedienbare Steuervorrichtung auf, mittels der die Leistung eines mittels Druckluft antreibbaren Schwingungserregers wahlweise vergrößerbar oder verringerbar ist. Die Steuervorrichtung umfaßt eine variable Drossel in der Zuführungsleitung für Druckluft und eine Einstellvorrichtung mit einem am Behandlungsinstrument angeordneten Drehring, mit dem die Drossel variierbar ist. Hierdurch läßt sich die Beaufschlagung des Schwingungserregers mit Druckluft im Sinne einer wahlweisen Vergößerung oder Verringerung steuern.

Ein medizinisches oder dentalmedizinisches Behandlungsinstrument der vorliegenden Art kann hinsichtlich seiner Leistungsabgabe störungsanfällig sein, obwohl der Schwingungserreger eine konstante und bestimmte Schwingleistung abgeben soll, um einen optimalen Schwingungsbetrieb zu erreichen. Der optimale Schwingungsbetrieb kann verhältnismäßig leicht durch unterschiedliche Leistungsgrößen beeinträchtigt werden, wobei auch kaum zu vermeidende Schwingungsresonanzen eine beeinträchtigende Wirkung auf das Schwingungsverhalten ausüben können. Dies gilt im besonderen für ein Behandlungsinstrument, dessen Schwingungserreger durch Druckluft antreibbar ist.

Der Erfindung liegt deshalb die Aufgabe zugrunde, ein Behandlungsinstrument der im Oberbegriff des Anspruchs 1 angegebenen Art so auszugestalten, daß eine konstante Schwingleistung erreicht wird.

Diese Aufgabe wird durch die Merkmale des Anspruchs 1 gelost.

Bei dieser erfindungsgemäßen Ausgestaltung ist dem Behandlungsinstrument ein Druckregler zugeordnet, der für einen konstanten am Schwingungserreger anstehenden Arbeitsdruck sorgt. Hierdurch ist gewährleistet, daß bei kaum zu vermeidenden Schwankungen des Betriebsdrucks, oder dann wenn das Behandlungsinstrument an Behandlungsplätzen eingesetzt wird, an denen unterschiedliche Betriebsdrücke herrschen (unterschiedliche Hersteller) die Schwingleistung gleich bleibt und somit ein optimaler Schwingungsbetrieb eingehalten werden kann.

Es ist besonders vorteilhaft, einen einstellbaren Druckregler vorzusehen, so daß unabhängig von der Höhe des Betriebsdrucks eine Einstellung der Größe der Leistung und eine jeweils optimale Leistungseinstellung möglich ist.

Die bekannten Behandlungsinstrumente sind bezüglich ihrer Schwingungsfunktion auf eine bestimmte Werkzeugart oder auf eine bestimmte Bearbeitung abgestimmt. Hierdurch ist der Verwendungsbereich eines solchen Behandlungsinstruments eingeschränkt

Die Erfindung bezweckt im weiteren, das Behandlungsinstrument dahingehend zu verbessern, daß es sich für einen breiteren Einsatzbereich eignet. Dies wird durch die Merkmale des Anspruchs 4 erreicht.

Bei dieser erfindungsgemäßen Ausgestaltung ist dem Schwingungserreger eine Steuervorrichtung zum Vergrößern und Verringern seiner abgegebenen Leistung zugeordnet, die eine Leistungseinstellung in Stufen oder stufenlos ermöglicht. Hierdurch ist es möglich, die Leistung des Behandlungsinstruments an unterschiedliche Bearbeitungen anzupassen. Das erfindungsgemäße Behandlungsinstrument eignet sich für einen breiten Bereich von Einsatzfällen, z.B. für Bearbeitungen mit unterschiedlichen Werkzeugen, insbesondere unterschiedlicher Größe und/oder Form und/oder Abtragsfähigkeit (Abrasivität), mit denen es wahlweise bestückbar ist. Außerdem ist beim Vorhandensein sowohl nur eines Werkzeugs als auch unterschiedlicher Werkzeuge die Leistung des Behandlungsinstruments an unterschiedlich intensive Bearbeitungen anpaßbar, nämlich an grobe und feine Bearbeitungen oder grobe, mittlere und feine Bearbeitungen, wobei diese Anpassungen unter Berücksichtigung gleicher Werkstoffe oder unterschiedlicher Werkstoffe des Werkzeugs und/oder des zu bearbeitenden Materials erfolgen kann. Dabei ermöglicht die erfindungsgemäße Ausgestaltung auch eine Anpassung des Behandlungsinstruments an unterschiedliche Bearbeitungsarten, z.B. zum Entfernen von Belägen, wie es bei einem Zahnsteinentfernungsgerät der Fall ist, oder zum Ausarbeiten einer Kavität, wie es z.B. bei der Präparation eines Zahnes der Fall ist.

In den Unteransprüchen sind Merkmale enthalten, die bei einfacher, kleiner und funktionssicherer Bauweise langlebig und kostengünstig herstellbar sind, eine handhabungsfreundliche Bedienung gewährleisten und eine einfache Montage bzw. Demontage gestatten.

In den Zeichnungsfiguren sind Ausführungs beispiele der Erfindung dargestellt. Es zeigen
- Fig.1: ein erfindungsgemäßes Behandlungsinstrument zur Behandlung von Körpergewebe oder einem Ersatzstoff im axialen Schnitt;
- Fig. 2: die in Fig. 1 mit X gekennzeichnete Einzelheit in vergrößerter Darstellung;
- Fig. 3: die Einzelheit gemäß Fig. 2 in vereinfachter schematischer Darstellung;
- Fig. 4: die in Fig. 1 mit Y gekennzeichnete Einzelheit, nämlich eine im vorderen Endbereich des Behandlungsinstruments angeordnete Haltevorrichtung für ein Werkzeug im axialen Schnitt;
- Fig. 5: einen Längsabschnitt eines erfindungsgemäßen Behandlungsinstruments in abgewandelter Ausgestaltung im axialen Schnitt;
- Fig. 6: den Längsabschnitt nach Fig. 13 in der Draufsicht.

Die Hauptteile des allgemein mit 1 bezeichneten Behandlungsinstruments sind ein längliches oder stabförmiges Handstück 2 mit einer Griffhülse 3, in der ein vorzugsweise längliches oder stabförmiges Schwingteil 4 schwingbar gelagert und durch einen Schwingungserreger 5 in Schwingungen versetzbar bzw. antreibbar ist, und eine Haltevorrichtung 6 für wenigstens ein Behandlungs-Werkzeug 7, das einen Werkzeugschaft 8 aufweist, der lösbar mit der Haltevorrichtung 6 verbindbar ist.

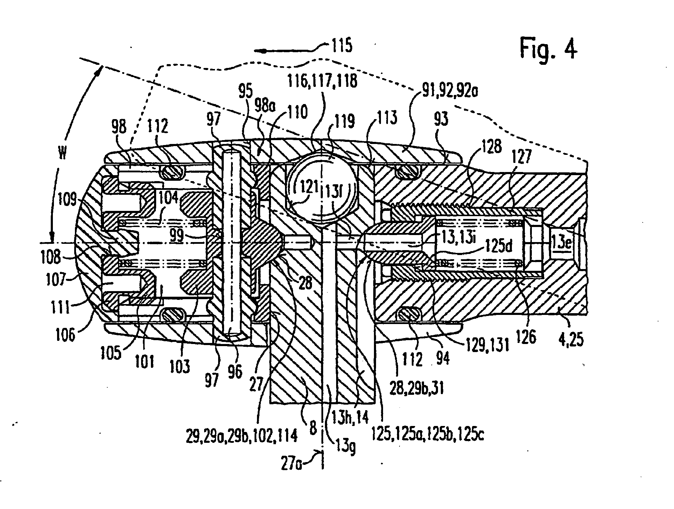

Es ist vorteilhaft, das Behandlungsinstrument 1 mit wenigstens einer, vorzugsweise mehreren sogenannten Medienleitungen auszugestalten, die der Zuführung von Antriebsenergie und Behandlungsmedien wie z.B. einer Behandlungsflüssigkeit und/oder Druckluft und der Zuführung von Licht zur Behandlungsstelle dienen. Bei der vorliegenden Ausgestaltung ist eine Lichtleitung 9 vorgesehen, die sich längs durch das Handstück 2 bis in dessen vorderen Endbereich erstreckt und an einer auf die Behandlungsstelle gerichteten Ausmündungsöffnung 11 ausmündet, und eine Beleuchtungseinrichtung 12 bildet. Außerdem ist eine sich längs durch das Handstück 2 erstreckende Leitung 13 für eine Behandlungs- oder Kühlflüssigkeit, hier Wasser, vorgesehen, die ebenfalls an einer im vorderen Endbereich des Handstücks 2 angeordneten und auf die Behandlungsstelle gerichteten Ausmündungsöffnung 14 (Fig. 4 und 7) ausmündet und eine Zuführungsvorrichtung bildet.

Ferner ist eine Zuführungsleitung 15 für Antriebsenergie vorgesehen, die sich längs durch das Handstück 2 bis zum Antrieb des Schwingungserregers 5 erstreckt. Bei der vorliegenden Ausgestaltung ist ein durch Druckluft antreibbarer Schwingungserreger 5 vorgesehen und die Zuführungsleitung 15 ist eine Druckluftleitung, die sich zum Schwingungserreger 5 erstreckt.

Das Handstück 2 ist durch eine Schnellkupplung oderdurch eine Schraub- oder Steckkupplung, insbesondere durch eine frei drehbare Steck-Drehkupplung 16, rückseitig mit einem angedeuteten Anschlußstück 17 lösbar verbindbar, das durch eine flexible Versorgungsleitung 18 mit einer nicht dargestellten Versorgungs- und Steuereinrichtung verbunden ist. Die Steck-Drehkupplung 16 ist bei der vorliegenden Ausgestaltung durch eine hohlzylindrische oder stufenhohlzylindrische Kupplungsausnehmung 16a und einen darin mit Bewegungsspiel einsteckbaren zylindrischen oder stufenzylindrischen Kupplungszapfen 16b gebildet, wobei bei der vorliegenden Ausgestaltung die Kupplungsausnehmung 16a im hinteren Bereich des Handstücks 2 angeordnet ist und der Kupplungszapfen 16b vom Anschlußstück 17 vorragt. Die Medienleitungen durchsetzen die Steck-Drehkupplung 16, so daß bei einem wahlweisen Trennen des Handstücks 2 vom Anschlußstück 17 die Medienleitungen selbsttätig unterbrochen werden. Die Lichtleitung 9 durchsetzt die Steck-Drehkupplung 16 koaxial. Dabei kann ein hinterer Lichtleitungsabschnitt sich koaxial in einer entsprechenden Aufnahmebohrung bis zum vorderen Endbereich des Kupplungszapfens 16b erstrecken an den sich mit einer Trennfuge ein vorderer Lichtleftungsabschnitt anschießt, der sich bis zur Ausmündungsöffnung 14 erstreckt. Der vordere Lichtleitungsabschnitt ist vorzugsweise durch einen Lichtleiter 9a aus Licht leitendem Material, wie z.B. Glas oder Kunststoff, gebildet, wobei es sich um einen flexiblen Lichtleiter oder um einen starren Lichtleiter in Form eines Formstücks handeln kann. Die Querschnittsform des Lichtleiters 9 ist vorzugsweise hinten rund und weiter vorne elipsenförmig. Der hintere Lichtleitungsabschnitt kann ebenfalls durch einen nicht dargestellten Lichtleiter oder durch eine elektrische Leitung mit einer im vorderen Endbereich des Kupplungszapfens 16b angeordnete Lampe 19 gebildet sein, die in einer vorderseitig offenen Ausnehmung vorzugsweise versenkt angeordnet ist.

Die beiden anderen Medienleitungen 13, 15 durchsetzen die hohlzylindrische Trennfuge 21 zwischen dem Kupplungszapfen 16b und der Wandung der Kupplungsausnehmung 16a Z-förmig radial von innen nach außen, wobei die jeweils in der Mantelfläche des Kupplungszapfens 16a und der Innenmantelfläche der Kupplungsausnehmung 16b vorhandenen Öffnungen in einer Ringnut 22 in der Mantelfläche oder in der Innenmantelfläche liegen. Hierdurch ist der Mediendurchgang in jeder Drehstellung der Steck-Drehkupplung 16 auch über 360° hinaus gewährleistet. Die Z-förmigen Durchdringungsstellen sind jeweils durch einen Dichtungsring 23, insbesonder einen O-Ring, abgedichtet, der zu beiden Seiten der Z-förmigen Durchdringungsstelle in einer Ringnut in der Mantelfläche des Kupplungszapfens 16b oder in der Innenmantelfläche der Kupplungsausnehmung 16a angeordnet ist. Rückseitig können am Anschlußstück 17 Anschlüsse, z.B. Anschlußhülsen 24, für in derflexiblen Versorgungsleitung 18 verlaufende Medienleitungsabschnitte angeordnet sein.

Die Haltevorrichtung 6 für das Werkzeug 7 ist im vorderen Endbereich des Schwingteils 4 angeordnet. Bei der vorliegenden Ausgestaltung ist das Schwingteil 4 ein stab- oder hülsenförmiger Körper, der in der vorderen Hälfte des Handstücks 2 angeordnet ist und einen Handstückschaft 25 bildet, der vom vorderen Ende des Handstücks 2 vorragt und sich gerade erstrecken kann oder bezüglich der Längsmittelachse 26 des Handstücks 2 einen spitzen Winkel W von etwa 10 bis 30°, insbesondere etwa 20°, einschließen kann. Die Haltevorrichtung 6 weist ein sich quer oder vorzugsweise rechtwinklig zum Handstückschaft 25 erstreckendes Steckloch 27 auf, das vorzugsweise ein Sackloch ist und somit nur einseitig offen ist, und in das der Werkzeugschaft 8 mit geringem Bewegungsspiel einsteckbar ist. Zur Sicherung des Werkzeugs 7 in der eingesteckten Stellung ist im Werkzeugschaft 8 eine Sicherungsausnehmung 28 vorgesehen, in die ein in der Längsrichtung des Handstückschaftes 25 verstellbares Sicherungsteil 29 hineinbewegbar ist, das zwischen seiner in die Sicherungsausnehmung 28 hineinbewegten Sicherungsstellung und einer herausbewegten, den Werkzeugschaft 8 freigebenden Freigabestellung verstellbar ist und vorzugsweise durch die Kraft einer Feder in seine Sicherungsstellung beaufschlagt ist. Bei der Sicherungsausnehmung 28 kann es sich um eine Ringnut handeln. Es können auch mehrere, auf den Umfang gleichmäßig verteilt angeordnete Kalotten vorgesehen sein, die vorzugsweise kegelförmige Einsenkungen mit einer zentralen Kern-Freibohrung aufweisen können.

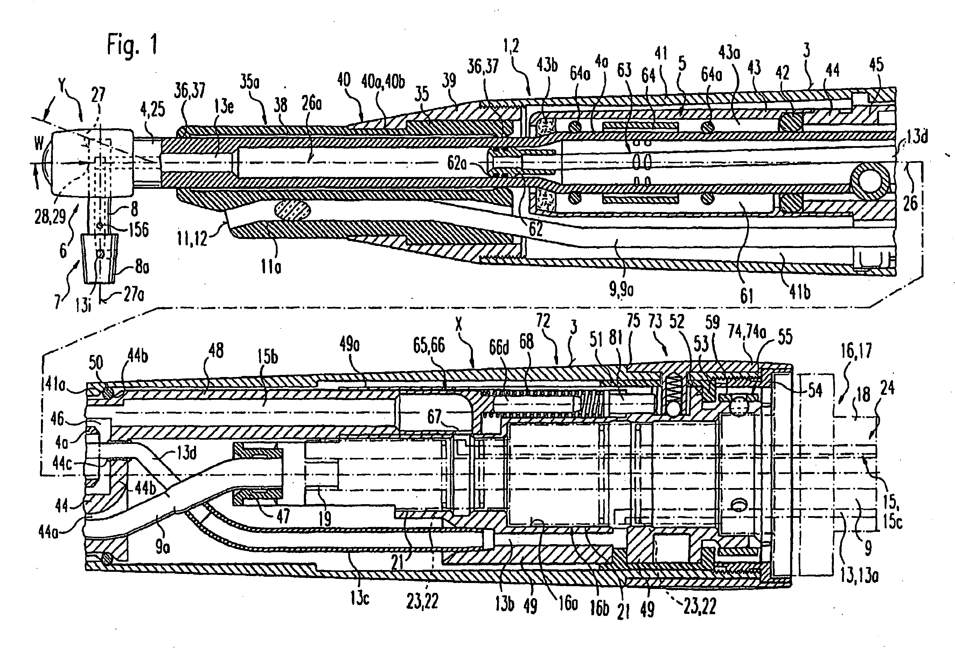

Der sich im Bereich des Handstücks 2 als Schwingstab 4a erstreckende Handstückschaft 25 ist gegen elastische Rückstellkräfte radial und vorzugsweise auch axial nachgiebig bzw. beweglich im Handstück 2 gelagert. Hierzu dient im vorderen Endbereich des Handstücks 2 eine Lagerhülse 35 aus elastischem Material, z.B. Kunststoff oder Gummi, in deren Durchgangsloch 36 der Schwingstab 4a mit umfangsseitiger Berührung aufgenommen ist, so daß er durch die Lagerhülse 35 zentriert ist. Bei der vorliegenden Ausgestaltung weist die Lagerhülse 35 in ihrem vorderen und hinteren Endbereich radial nach innen vorspringende, vorzugsweise im Querschnitt gerundete Lagerwülste 37 auf, zwischen denen ein den Schwingstab 4a umgebender Ringspalt 38 angeordnet ist. Die Lagerhülse 35 kann in einer Hülsenkappe 39 fest angeordnet sein, die mit einem Hülsengehäuse 41 fest verbunden, z.B. darin eingeschraubt ist, wie es Fig. 1 zeigt, und die umfangsseitig miteinander stufenlos abschließen und die Griffhülse 3 bilden.

Die Lagerhülse 35 überragt das Hülsengehäuse 41 bzw. die Hülsenkappe 39 und bildet damit einen Schutzmantel 35a für den Handstückschaft 25, der sich bis zur oder bis in die Nähe der Haltevorrichtung 6 bzw. ein noch zu beschreibendes Betätigungsglied 92a erstreckt. Zur axialen Fixierung der Lagerhülse 35 und oder des Schutzmantels 35a ist eine formschlüssige Verbindung 40 mit einer Ringnut 40a und einem darin einfassenden Ringzapfen 40b vorgesehen, der aufgrund des elastisch verformbaren Materials beim Einstecken selbsttätig einrastet. Durch den vorragenden elastischen oder weichelastischen Schutzmantel 35a werden bei der Behandlung aus der Vibration resultierende Beeinträchtigungen bei der Berührung des Werkzeugschaftes mit benachbarten Körperteilen, z.B. Zähnen, vermieden. Im Rahmen der Erfindung kann der Schutzmantel auch als separates Bauteil nur auf dem Werkzeugschaft 25 angeordnet und fixiert sein, z. B. darauf elastisch klemmend aufgeschoben oder mittels einer entsprechenden formschlüssigen Verbindung 40 darauf fixiert sein.

Im hinteren Bereich des Schwingstabs 4a ist die radial und axial elastisch nachgiebige Lagerung für den Schwingstab 4a durch einen Lagerring 42 aus elastisch verformbaren Material wie Kunststoff oder Gummi, insbesondere einen O-Ring, gebildet, in dem der Schwingstab 4a mit außenseitigem Kontakt sitzt, wobei der Lagerring 42 in einer den Schwingstab 4a mit radialem Ringraum 43a umgebenden Hülse 43 oder in einen sich von letzterer nach hinten erstreckenden Lagerteil 44 aufgenommen und gelagert ist, die bzw. das im Hülsengehäuse 41 radial und axial unbeweglich oder vorzugsweise gegen eine Rückstellkraft beweglich gelagert ist, insbesondere von hinten eingeschoben ist und mit einervorderseitigen Schulterfläche an einer rückseitigen Schulterfläche des Hülsengehäuses 41 anliegt. Das Lagerteil 44 weist einen vorderseitigen Hülsenansatz 45 auf, der den Schwingstab 4a mit radialem Abstand umgibt, wobei das hintere Ende des Schwingstabs 4a in einem Freiraum 46 des Lagerteils 44 endet. Der vordere Hülsenansatz 45 ist in einander überlappender Anordnung mit der Hülse 43 verbunden, vorzugsweise schließend auf- oder eingesteckt und durch eine Dichtung abgedichtet, die hierdurch den Lagerring 42 gebildet ist.

Der Schwingstab 4a kann mit den zugehörigen Lagerelementen koaxial zur Längsmittelachse 26 angeordnet sein. Bei der vorliegenden Ausgestaltung ist die Längsmittelachse 26a des Schwingstabes 4a bezgl. der Längsmittelachse des vorderen Endbereichs der Griffhülse 3 und der Lagerhülse 35 zur dem seitlich angeordneten Werkzeugkörper 8a abgewandten Seite hin versetzt und somit exzentrisch angeordnet, wobei die Ausmündungsöffnung 11 und der zugehörige Durchgangskanal 11a, in dem der Lichtleiter 9a aufgenommen ist, bezgl. des Schwingstabs 4a zur dem Werkzeug zugewandten Seite hin angeordnet ist, hier in der entsprechend exzentrischen Lagerhülse 35. Das rückseitige Ende des Lichtleiters 9a ist mittels einer Lagerhülse 47 in einer Verlängerung der Kupplungsausnehmung 16a angeordnet, wobei sich der Lichtleiter 9a durch einen exzentrischen Durchgangskanal 44a im Lagerteil 44 als seitlich bogenförmig ausgeformtes Teil nach vorne erstreckt. Dem Lichtleiter 9a gegenüberliegend weist das Lagerteil 44 einen exzentrischen rückseitigen Rohrstutzen 48 auf, an das sich rückseitig ein Hülsenvorsprung 49a einer Kupplungshülse 49 anschließt, die allein oder mit dem Lagerteil 44 als zusammengesetztes Bauteil radial allseitig und axial gegen eine Rückstellkraft beweglich im Hülsengehäuse 41 gelagert ist. Hierzu dient wenigstens ein die Kupplungshülse 49 umgebender Lagerring 51 aus elastisch verformbarem Material, der die Kupplungshülse 49 aufgrund seiner elastischen Rückstellkraft zentriert und radial elastisch nachgiebig lagert. Vorzugsweise weist der Lagerring 51 einen eine rückseitige Schulterfläche 52 hintergreifenden Innenflansch 53 auf, der die Kupplungshülse 49 auch bezgl. axialen nach hinten gerichteten Bewegungen elastisch nachgiebig lagert. Bei der vorliegenden Ausgestaltung ist die durch die Kupplungshülse 49 und das Lagerteil 44 gebildete Baueinheit zwischen dem Innenflansch 53 und dem Lagerring 42 in beide axiale Richtungen elastisch nachgiebig gelagert. Zusätzlich kann zwischen zugehörigen Schulterflächen 41a, 44b, hier zwischen einer Innenschulter des Hülsengehäuses 41 und einer Außenschulter des Lagerteils 44, ein axial und vorzugsweise auch diametral wirksamer Lagerring 50 aus elastisch nachgiebigem Material angeordnet sein, wobei aufgrund der elastischen Rückstellkraft der elastisch nachgiebigen Lagerelemente eine Mittenzentrierung gewährleistet ist und zwar sowohl axial als auch diametral. Der Lagerring 51 ist in axialer Richtung formschlüssig fixiert, z.B. dadurch, daß sein Innenflansch 53 an einer nach vorne gerichteten Schulterfläche einer in das hintere Ende des Hülsengehäuses 41 eingeschraubte Gewindehülse 54 anliegt.

Im hinteren Bereich der Steck-Drehkupplung 16 ist eine manuell überdrückbare Verrastungsvorrichtung 55 zum überdrückbaren Verrasten in der Kupplungsendstellung vorgesehen. Bei der vorliegenden Ausgestaltung weist die Verrastungsvorrichtung 55 eine oder mehrere auf den Umfang in radialen Löchern der Kupplungshülse 49 verteilt angeordnete, vorzugsweise durch Kugeln gebildete Verrastungselemente, gebildet, die soweit in die Löcher eintauchen können, daß die radial inneren Enden der Verrastungselemente in eine Ringnut im Kupplungszapfen 16b einrasten können. Beim manuellen Überdrücken der Steck-Drehkupplung werden die Verrastungselemente selbsttätig gegen eine sie nach innen radial beaufschlagende Feder in Form eines Federrings 59 aus der Ringnut herausgedrückt. Die Löchersind an ihren inneren Enden verjüngt, so daß das oder die Verrastungselemente, vorzugsweise Kugeln bei entferntern Kupplungszapfen 16b nicht nach innen herausfallen können.

Die Medienleitungen erstrecken sich im Anschlußstück 17 als z.B. achsparallele Kanäle 13a, 15a, die sich in der zugehörigen Durchgangs-Querebene in Form von Winkelkanälen im Kupplungszapfen 16b und in der Kupplungshülse 49 fortsetzen, wobei die eine Durchgangs-Querebene E1 bezüglich der anderen Durchgangs-Querebene E2 der Druckluftleitung axial versetzt ist und die Winkelkanäle in der Kupplungshülse 49 mit 13b, 15b bezeichnet sind. An den Winkelkanal 13b der Leitung 13 für Behandlungsflüssigkeit schließt sich ein Rohr oder Schlauch 13c an, der stromab mit einem weiteren Rohr oder Schlauch 13d verbunden ist, der eine Rückwand 44b des Lagerteils 44 in einer Durchführungshülse 44c abgedichtet durchsetzt und sich im Hohlraum 61 des hülsenförmigen Schwingstabs 4a bis in dessen mittleren Bereich erstreckt, in dem eine Endhülse 62 mittels einem oder zwei Dichtungsringen 62a abgedichtet im hülsenförmigen Schwingstab 4a sitzt und dicht mit dem Rohr 13d verbunden ist, insbesondere darauf aufgesetzt ist.

Dem Schwingungserreger 5 ist eine Steuervorrichtung zum Verringern oder Vergrößern seiner Leistung zugeordnet. Hierdurch läßt sich seine Leistungsfähigkeit bzw. die Intensität der Vibration bzw. Schwingungen und die Größe der Amplituden wahlweise verringern oder vergrößern und somit einstellen. Dabei kann ein im einzelnen noch zu beschreibender Leistungsregler 71 zum automatischen Regeln einer vorzugsweise konstanten Leistung und/oder eine vorzugsweise manuell einstellbare Steuervorrichtung 72 vorgesehen sein, mit dem bzw. mit der die Leistung oder die dem Schwingungserreger 5 zuführbare Antriebsenergiemenge veränderlich und verringerbar und vergrößerbar ist und selbsttätig oder durch eine manuelle Einstellvorrichtung 73 in Stufen oder stufenlos einstellbar ist. Hierdurch kann die Leistung des Behandlungsinstruments an die zu verrichtende Arbeit, z.B. Grob- und Feinarbeit oder Grob-, Mittel- und Feinarbeit oder an unterschiedliche Arten der Behandlung und/oder an hinsichtlich Form und/oder Größe und/oder grober und feiner oder grober, mittlerer und/oder feiner Wirksamkeit unterschiedliche Werkzeuge 7 angepaßt werden.

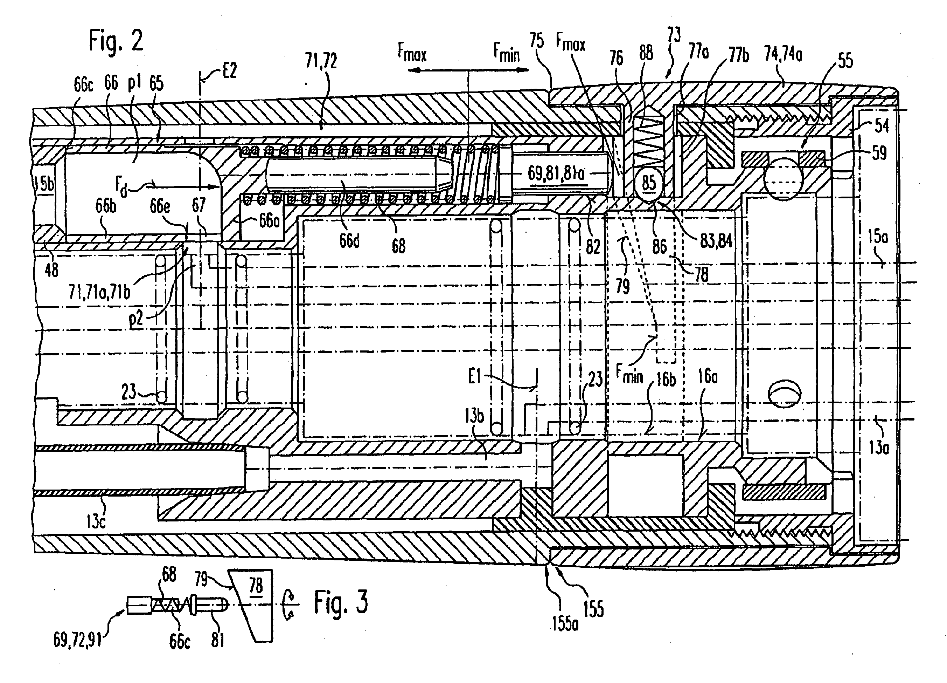

Die manuell betätigbare Einstellvorrichtung 73 weist ein Einstellglied 74 auf, das von außen manuell zugänglich und axial oder in Umfangsrichtung verstellbar ist und in Antriebsverbindung mit der Steuervorrichtung 72 steht oder eine Steuervorrichtung bildet. Bei der vorliegenden Ausgestaltung ist das Einstellglied 74 eine Einstellhülse 74a die das Handstück 2 vorzugsweise in dessen hinteren Bereich umgibt und insbesondere in einer eine Ringführung bildenden Ringausnehmung 75 versenkt angeordnet und axial oder vorzugsweise in Umfangsrichtung verstellbar ist. Bei der vorliegenden Ausgestaltung ist die Ringausnehmung 75 rückseitig durch eine Schulterfläche der Einschraubhülse 54 begrenzt. Von der Innenmantelflächeder Einstellhülse 74a ragt ein Tragsteg 76 radial einwärts vor, der sich über einen Teil des Umfangs erstreckt und Schlitze 77a, 77b im Hülsengehäuse 41 und den davon radial einwärts angeordneten Bauteilen, hier in der Kupplungshülse 49 und im Lagerring 51, mit Bewegungsspiel durchfaßt und an seinem inneren Ende ein sich über einen Abschnitt des Umfangs erstreckendes Segment 78 trägt, das an einer Seite eine Schräg- oder Kurvenfläche 79 aufweist, die mit einem Verstellglied 81 zusammenwirkt, das mit der Steuervorrichtung 72 in Wirkverbindung steht, wie es Fig.3 schematisch und Fig.1 und 2 als Ausführungsbeispiel zeigen. Die Schräg- oder Kurvenfläche 79 kann vorderseitig und rückseitig am Segment 78 angeordnet sein. Bei der vorliegenden Ausgestaltung, bei der das Einstellglied 74 im hinteren Endbereich des Handstücks 2 befindet, ist die Schräg- oder Kurvenfläche 79 vorderseitig angeordnet, und das Verstellglied 81 ist ein achsparallel in einer Führung 82 verschiebbar gelagerter Verstellstift 81a, der durch die Kraft einer Feder, hier einer Druckfeder gegen die Schrägoder Kurvenfläche 79 beaufschlagt ist und diese somit bei einer Hin- und Herverdrehung des Verstellglieds 74 abzugreifen vermag. Die Führung 82 kann durch eine axparallele Bohrung in der drehgesichert gelagerten Kupplungshülse 49 gebildet sein.

Die Einstellvorrichtung 73 kann in Stufen oder stufenlos einstellbar sein: In beiden Fällen ist es vorteilhaft, der Einstellvorrichtung 73 eine Feststellvorrichtung 83 zuzuordnen, die ein Feststellen der Einstellvorrichtung 73 in der jeweils eingestellten Position ermöglicht und dadurch eine unbeabsichtigte Verstellung verhindert. Hierbei kann es sich um eine Bremsvorrichtung handeln, die aufgrund ihrer Schwergängigkeit eine unbeabsichtigte Verstellung des Verstellglieds 74 verhindert. Bei der vorliegenden Ausgestaltung ist eine überdrückbare Verrastungsvorrichtung 84 mit einem vorzugsweise durch eine Kugel gebildeten, radial beweglich geführten Verrastungselement 85 vorgesehen, das beim Drehen der Einstellhülse 74a in Kalotten 86 manuell überdrückbar ein- und ausrastbar ist, die auf einer Außenmantelfläche der Kupplungshülse 49 in Umfangsrichtung hintereinaneinanderliegend angeordnet sind, hier im Grund der Nut bzw. des Schlitzes 77b. Das Verrastungselement 85 kann in einer radialen Bohrung im Tragsteg 76 radial verschiebbar gelagert sein und durch eine in der Bohrung angeordnete Druckfeder 88 gegen die Kalotten 86 vorgespannt sein. Das Verstellglied 81 wirkt auf den in seiner Leistungsfähigkeit einstellbaren Schwingungserreger 5, so daß dessen Leistung mit dem Einstellglied 74 wahlweise zu vergrößern oder zu verringern ist.

Beim vorliegenden Ausführungsbeispiel, bei dem der Schwingungserreger 5 pneumatisch antreibbar bzw. erregbar ist, kann die Steuervorrichtung 72 ein Steuerventil 65 aufweisen zwecks Steuerung des am Schwingungserreger 5 wirksamen Druckes p1. Hierbei kann das Einstellglied 74 mittelbar oder unmittelbar mit einem Ventilschieber 66 verbunden sein, der die Größe einer Ventilöffnung 67 in Abhängigkeit von der Einstellung des Einstellglieds 74 steuert. Die Ventilöffnung 67 befindet sich in der Zuführungsleitung 15, hier stromab vom Kupplungszapfen 16b im Bereich des Winkelkanals 15b. Zur Verringerung der Schwingungsleistung wird der Ventilschieber 66 mit der Einstellvorrichtung 73 im Sinne einer Verkleinerung der Ventilöffnung 67 verschoben, beim vorliegenden Ausführungsbeispiel nach vome, so daß die Ventilöffnung 67 den am Schwingungserreger 5 anstehenden Druck p1 im Sinne einer verstellbaren Drossel verringert. Zur Vergrößerung der Leistung wird die Ventilöffnung 67 im umgekehrten Sinnevergrößert, wodurch ein größerer anstehender Druck p1 eingestellt wird. Die Rückführung des Einstellglieds 74 kann durch eine Rückholfeder erfolgen, die das Einstellglied 74 gegen die Schräg- oder Kurvenfläche 79 vorspannt.

Es ist vorteilhaft, zusätzlich oder anstelle der Steuervorrichtung 72 zur Einstellung des anstehenden Druckes p1 eine automatische Druck-Regelvorrichtung 71a mit einem Druckregelventil 71b vorzusehen, die bzw. das unabhängig vom vorhandenen Betriebsdruck p2 im Zuführungsleitungsabschnitt 15a einen im wesentlichen konstanten wirksamen Druck p1 einstellt. Hierdurch werden auch bei beträchtlichen Toleranzen des wirksamen Druckes p1 im wesentlichen gleiche Arbeitsbedingungen und eine etwa vergleichbare Leistung bzw. Intensität des Werkzeugs 7 beim Anschluß des Behandlungsinstruments an Versorgungsleitungen 18 und Versorgungseinrichtungen mit unterschiedlichen Betriebsdrücken p2, insbesondere von unterschiedlichen Herstellern, erreicht.

Sowohl beim Vorhandensein eines Steuerventils 65 als auch eines Regelventils 71b ist der Ventilschieber 66 in der Zuführungsleitung 15, hier im achsparallelen Zuführungsleitungsabschnitt 15b, angeordnet, vorzugsweise darin längsverschiebbar gelagert, wobei die Ventilköffnung 67 durch ein Stirnfläche des Ventilschiebers 66 gesteuert werden kann. Der Ventilschieber 66 kann auch eine topfförmige Form aufweisen, wobei die Ventilöffnung 67 in der Umfangswand der Topfform angeordnet sein kann.

Beim vorliegenden Ausführungsbeispiel gemäß Fig. 1 bis 3 sind eine Steuervorrichtung 72 oder eine manuelle Einstellvorrichtung 73 in Kombination mit einem Druckregelventil 71 b vorgesehen. Der Ventilschieber 66 wird auf seiner einen Stirnseite vom wirksamen Druck p1 beaufschlagt und auf seiner anderen Stirnseite mittels einer Druckfeder 68 entgegengesetzt in seine Offenstellung vorgespannt. Bei der vorliegenden Ausgestaltung ist der Ventilschieber 66 eine runde oder unrunde topfförmige Hülse mit einer Bodenwand 66a an seinem dem Schwingungserreger 5 abgewandten Ende. Die Ventilöffnung 67 ist in der radial innenliegenden Umfangswand 66b mit dem radialen Abschnitt des Zuführungsleitungsabschnitts 15b zusammenwirkend angeordnet. In der Offenstellung ist der Ventilschieber 66 durch die Feder 68 gegen einen Anschlag 66c gespannt, hier den Rohrstutzen 48. Vom Ventilschieber 66 erstreckt sich ein Federdom 66d nach hinten, auf dem die Druckfeder 68 sitzt und gegen ein rückseitiges Widerlager 69, hier gegen das Verstellglied 81, abgestützt ist.

Im Funktionsbetrieb strömt die Druckluft vom Zuführungsleitungsabschnitt 15b durch den am rückseitigen Ende vorhandenen Ringspalt in den Hohlraum 61 des hülsenförmigen Schwingstabs 4a, der im mittleren Bereich und vor der Endhülse 62 ein oder mehrere radiale und/oder sekantiale Durchströmlöcher 63 aufweist, in deren Bereich der Schwingstab 4a mit radialem Bewegungsspiel von einer Taumel- oder Vibrationshülse 64 umgeben ist, die mit axialem Bewegungsspiel zwischen zwei Anschlägen 64a angeordnet ist, die jeweils durch einen Ring aus elastischem Material, z.B. einen O-Ring, gebildet sein können, der in einer Ringnut im Schwingstab 4a sitzt. Während der Durchströmung des Spalts zwischen dem Schwingstab 4a und der Vibrationshülse 64 wird letztere in Schwingung versetzt, die sie durch mechanisches Anschlagen auf den Schwingstab überträgt. Der Spalt ist so groß bemessen, daß die Vibrationshülse 64 nicht gegen die Innenwandung der sie umgebenden Hülse 43 schlägt. Aus dem Ringraum 43a strömt die Luft bzw. Abluft durch einen zwischen dem Schwingstab 4a und dem vorderen Endbereich der Hülse 43 angeordneten luftdurchlässigen Stützring 43b, der perforiert sein kann und aus elastischem oder nachgiebigem Material wie Kunststoff oder luftdurchlässigem Material wie z.B. Filz bestehen kann, in den freien Hohlraum 41b des Hülsengehäuses 41 und durch eine geeignete insbesondere hintere Öffnung (nicht dargestellt) im Hülsengehäuse 41 ins Freie oder z.B. durch den Kanal 44a und durch einen geeigneten Abführungskanal im Kupplungszapfen 16b zurück ins Freie. Der Stützring 43b ist axial fixiert, hier zwischen einer gegebenenfalls kegelförmigen Schulterfläche am Schwingstab 4a und einem Innenflansch der Hülse 43.

Die Hülse 43 bildet in Verbindung mit dem Stützring 43b ein Innengehäuse bzw. eine Kapselung des Schwingungserregers 5, wodurch eine wesentliche Geräuschminderung auf einfache und kostengünstige Weise erreicht wird.

Die Ventilöffnung 67 ist eine verstellbare Drossel, die einen Druckabfall erzeugt, der den wirksamen Druck p1 bestimmt. Wenn der Betriebsdruck p2 größer oder kleiner als ein bestimmter Wert ist, ändert sich zunächst auch der Druck p1, der die den Ventilschieber 66 gegen die Feder 68 beaufschlagende Druckkraft Fd erzeugt, wobei die Stellung des die Größe der Ventilöffnung 67 bestimmenden Öffnungsrandes 66e und somit auch die Größe der Drossel, die den wirksamen Druck p1 erzeugt, durch das Gleichgewicht der Federkraft und der Druckkraft Fd bestimmt ist. Im Funktionsbetrieb nimmt der Ventilschieber 66 eine von der dargestellten Anschlagstellung (Außerfunktionsstellung) entfernte Funktionsstellung ein, in derz.B. bei einem mittleren Betriebsdruck p2 die Ventilöffnung 67 eine Größe im mittleren Bereich einnimmt. Bei einem niedrigeren Betriebsdruck p2 wird der Ventilschieber 66 in eine Gleichgewichtsstellung (hier nach links) verschoben, in der er eine größere Ventilöffnung 67 steuert. Bei einem vergrößerten Betriebsdruck p2 nimmt der Ventilschieber 66 eine (hier nach rechts verschobene) Gleichgewichtsstellung ein, in der er eine kleinere Ventilöffnung 67 steuert. Aufgrund dieser Funktion regelt das Regelventil 71b bei in einem bestimmbaren Druckbereich unterschiedlichen Betriebsdrücken automatisch einen im wesentlichen konstanten anstehenden Druck p1. Diese Druckregelung funktioniert sowohl dann, wenn eine Einstellvorrichtung 73 oder eine sonstige Steuervorrichtung 72 fehlt, als auch dann, wenn-wie beim vorliegenden Ausführungsbeispiel - der Druck- bzw. Leistungsregler 71 in Kombination mit der Einstellvorrichtung 73 oder einer sonstigen Steuervorrichtung 72 angeordnet ist oder auch dann, wenn - wie beim vorliegenden Ausführungsbeispiel - die manuelle Einstellvorrichtung 73 oder eine sonstige Steuervorrichtung 72 auf das Druckregelventil 71b bzw. auf dessen Ventilschieber 66 einwirkt. Vorzugsweise ist eine gemeinsame Ventilöffnung 67 für das Druckregelventil 71b und die manuelle Einstellvorrichtung 73 oder eine sonstige Steuervorrichtung 72 vorhanden. Hierdurch wird der Bau- und Herstellungsaufwand beträchtlich verringert und die Bauweise vereinfacht.

Beim vorliegenden Ausführungsbeispiel wird mittels der Einstellvorrichtung 73 die Vorspannkraft der Ventilfeder 68 verändert und zwar für hohen Leistungsbedarf vergrößert und für niedrigeren Leistungsbedarf verringert. Bei höherem Leistungsbedarf wird somit das Verstellglied 81 in Richtung auf das Steuer- oder Regelventil 65, 71b verschoben und bei geringerem Leistungsbedarf vom Steuer- oder Regelventil 65, 71b entfernt d.h. nach hinten verschoben, was durch die Bezugszeichen Fmin und Fmax verdeutlicht ist. Beim vorliegenden Ausführungsbeispiel wird mittels der Einstellvorrichtung 73 die Leistung des Leistungsreglers 71 im Sinne einer Vergrößerung oder Verringerung der am Werkzeug 7 abgebbaren Leistung verändert und zwar unabhängig von der Größe des Betriebsdruckes p2.

Hierdurch läßt sich das Behandlungsinstrument 1 nicht nur an unterschiedliche Behandlungsmethoden sondern auch an Werkzeuge 7 unterschiedlicher Form und/oder Größe und/oder abrasiver Abtragungsfähigkeit, z.B. grob und fein oder grob, mittel und fein, anpassen, wobei auch eine Abstimmung unter Berücksichtigung von Werkzeugen 7 unterschiedlicher Massen und/oder Formen möglich ist. Außerdem lassen sich mit der Einstellvorrichtung 73 einander störende Schwingungszustände durch Veränderung der Schwingungsamplituden weitgehend ausschalten.

Die Hauptrichtung der Schwingungs-Aplitude ist im wesentlichen quer zum Schwingteil 4a und somit im wesentlichen in der Längsrichtung des Werkzeugs 7 gerichtet. Aufgrund der radialen und axialen elastisch nachgiebigen Lagerung des Schwingteils 4a stellen sich jedoch unter anderem durch Resonanzen hervorgerufene räumlich Schwingungen ein, so daß das Werkzeug 7 auch in Querrichtung abrasiv wirksam ist.

Beim vorliegenden Ausführungsbeispiel weist der Oszillations- bzw. Schwingungsantrieb eine Frequenz im Schall- oder Ultraschallbereich von etwa 4 bis 8 kHz, vorzugsweise etwa 6 kHz, auf, wobei sich im Bereich des Werkzeugs 7 eine Amplitude der räumlichen Bewegungen von etwa 0,05 mm bis 0,2 mm, insbesondere etwa 0,1 mm ergibt.

Das erfindungsgemäße Behandlungsinstrument eignet sich deshalb besonders gut für unterschiedliche Werkzeuge 7, die dem Behandlungsinstrument als Werkzeug-Sortiment zugeordnet sind und sich aufgrund unterschiedlicher Form und/oder Größe und/oder Zweckbestimmung voneinander unterscheiden.

Dem Behandlungsinstrument 1 können zwecks Durchführung unterschiedlicher Bearbeitungen unterschiedliche Werkzeuge 7 zugeordnet sein, mit denen es wahlweise bestückbar ist. Hierbei kann es sich um Werkzeuge 7 von z.B. unterschiedlicher Form und/oder Masse und/oder Beschaffenheit der abrasiven Arbeitsfläche, z. B. grob, mittel, fein, und/oder Werkzeuge 7 handeln, die für eine Zuführung eines Behandlungsmediums, z.B. Kühlflüssigkeit, eingerichtet sind oder nicht. Für solche unterschiedlichen Werkzeuge können unterschiedliche Leistungsanforderungen bestehen, um eine optimale Funktion oder Arbeitsleistung zu erreichen. Bei Werkzeugen 7 unterschiedlicher Masse kann dies dadurch bedingtsein, daß die Massenschwingung bei einer Leistungseinstellung spezifischer Größe besonders günstig ist, z.B. unter Berücksichtigung von Schwingungsresonanzen. Bei Werkzeugen 7 unterschiedlicher Beschaffenheit ihrer Arbeitsflächen, wie z. B. grob, mittel oder fein, ist ebenfalls eine Leistungseinstellung spezifischer Größe zwecks Verbesserung der Leistungsfähigkeit vorteilhaft. Außerdem ist bei diese Kriterium zu berücksichtigen, daß die Arbeitsflächen dazu neigen können, sich mit Spänen zuzusetzen und auch deshalb eine spezifische Leistungseinstellung vorteilhaft ist. Eine Leistungseinstellung in spezifischer Größe und ist auch bei Werkzeugen 7 vorteilhaft, die mit einer Kühlflüssigkeit oder trocken arbeiten. Es ist deshalb vorteilhaft, und dient einer einfacheren Handhabung, wenn am Behandlungsinstrument 1 Markierungen 155 für das Einstellglied 74 so angeordnet sind, daß bei Positionsübereinstimmung des Einstellgliedes 74 im Sinne der oder einer bestimmten Markierung eine Leistung eingestellt ist, die für ein zugeordnetes Werkzeug 7 mit einer korrespondierenden Markierung 156 vorteilhaft ist. Es kann sich um farbliche oder andere Markierungen, z.B. Symbole, handeln, denen jeweils ein zugehöriger Index 155a zur Postionsübereinstimmung auf dem Einstellweg am Behandlungsinstrument zugeordnet ist. Bei der vorliegenden Ausgestaltung können eine oder mehrere Markierungen 155 und ein oder mehrere zugehörige Indexe 155a am Einstellglied 74 und an der Griffhülse bzw. dem Hülsengehäuse 41 angeordnet sein, wie es vereinfacht dargestellt ist, z.B. in Form einer Skala, wobei an dem einen Teil die Markierungen 155 und an dem anderen Teil der wenigstens eine Index 155a angeordnet ist. An den Werkzeugen 7 kann jeweils die korrespondierende Markierung 156, z.B: am Werkzeugschaft 8 in der Nähe des Werkzeugkopfes, angeordnet sein. Unterschiedliche Farben eignen sich besonders gut, um ein Markierungs-Paar 155, 155a,156 von einem anderen Markierungs-Paar 155, 155a, 156 zu unterscheiden, wobei miteinander korrespondierende Markierunden 155, 155a, 156 vorzugsweise die gleiche Farbe aufweisen.

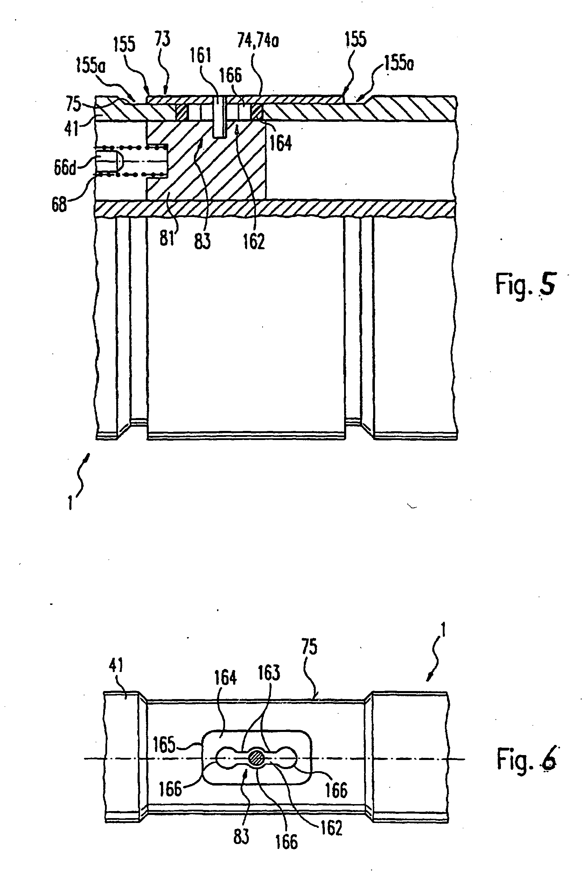

Beim Ausführungsbeispiel nach Fig. 5 und 6, bei dem gleiche oder vergleichbare Teile mit gleichen Bezugszeichen versehen, weist die Einstellvorrichtung 73 ein Einstellglied 74 vorzugsweise ebenfalls in Form einer Einstellhülse 74a auf, die in der entsprechend länger bemessenen Nut 75 axial verschiebar und in der jeweiligen Einstellposition durch die Feststellvorrichtung 83 stufenlos oder in Stufen feststellbar ist. Bei dieser Ausgestaltung ist das Verstellglied 81 durch einen radialen Verbindungsstift 161 starr mit dem Einstellglied 74 verbunden, wobei der Verbindungsstift 161 eine längs verlaufende Einstellnut 162 im Hülsengehäuse durchsetzt und wobei die längs gerichteten Nutwände 163 den Verbindungsstift 161 so stark zwischen sich klemmen, daß der Verbindungsstift nur mit einem manuellen Kraftaufwand axial verschiebbar ist, gegen eine unbeabsichtigte Verschiebung jedoch durch die Klemmwirkung der Nutwände 163 festgestellt ist. Hierzu kann die Einstellnut 162 in einem Einsatzteil 164 aus elastisch verformbaren Material, z.B. Kunststoff, ausgebildet sein, das in einer entsprechenden Ausnehmung 165 im Hülsengehäuse 41 sitzt. Falls bestimmte Einstellpunkte verwirklicht sein sollen, können in einer oder in beiden Nutwänden 163 Rastausnehmungen 166 angeordnet sein, in die der Verbindungsstift 161 einzurasten vermag. Bei der vorliegenden Ausgestaltung sind drei Raststellen axial hintereinander angeordnet. Um dies zu verdeutlichen, ist in Fig. 6 der dargestellte Längsabschnitt des Behandlungsinstruments 1 ohne die Einstellhülse 74a dargestellt.

Die Markierungen 155, 155a und 156 können bei dieser Ausgestaltung an der Vorderkante oder Hinterkante des Einstellglieds 74 und der daneben befindlichen Mantelfläche des Hülsengehäuses 41 angeordnet sein.

## Patentansprüche

1. Medizinisches oder dentalmedizinisches Behandlungsinstrument (1) zur spanabhebenden Bearbeitung von Körpergewebe oder einem Ersatzstoff mit einem abrasiven Werkzeug (7), bestehend aus einem Handstück (2) in dessen vorderem Endbereich ein das Werkzeug (7) tragendes Schwingteil (4) gelagert ist, das durch einen in dem Handstück (2) befindlichen Schwingungserreger (5) in Schwingungen versetzbar ist, wobei das Handstück (2) an seinem hinteren Endbereich mit einer flexiblen Versorgungsleitung (18) verbunden oder verbindbar ist, die sich von einer Versorgungseinrichtung erstreckt, und wobei der Schwingungserreger (5) durch Druckluft antreibbar bzw. erregbar ist, die durch eine sich durch das Handstück (2) erstreckende Zuführungsleitung (15b) zugeführt wird,
**dadurch gekennzeichnet,**
**daß** in der Zuführungsleitung (15b) ein Druckregler (71a) vorgesehen ist, der auch beim Vorhandensein unterschiedlich hoher Betriebsdrücke (p2) einen im wesentlichen konstanten, am Schwingungserreger (5) anstehenden Arbeitsdruck (p1) regelt,
wobei der Druckregler (71a) und damit die Leistung des Schwingungserregers (5) einstellbar ist.

2. Medizinisches oder dentalmedizinisches Behandlungsinstrument (1) nach Anspruch 1,
**dadurch gekennzeichnet,**
**daß** der Druckregler (71a) ein Druckregelventil (71b) mit einem Ventilschieber (66) umfaßt, der vom Arbeitsdruck (p1) gegen die Kraft einer Feder (68) vorzugsweise axial verstellbar gelagert ist, und bei größerem Betriebsdruck (p2) eine kleinere Ventilöffnung (67) und bei kleinerem Betriebsdruck (p2) eine größere Ventilöffnung (67) steuert.

3. Medizinisches oder dentalmedizinisches Behandlungsinstrument (1) nach einem der vorherigen Ansprüche,
**dadurch gekennzeichnet,**
**daß** eine Steuervorrichtung (72) vorgesehen ist, mit der die Leistung vergrößerbar oder verringerbar ist.

4. Medizinisches oder dentalmedizinisches Behandlungsinstrument nach Anspruch 3,
**dadurch gekennzeichnet,**
**daß** eine Einstellvorrichtung (73) mit einem manuell verstellbaren Einstellglied (74) vorgesehen ist, die auf die Steuervorrichtung (72) einwirkt oder die Steuervorrichtung (72) bildet.

5. Medizinisches oder dentalmedizinisches Behandlungsinstrument nach Anspruch 3 oder 4,
**dadurch gekennzeichnet,**
**daß** der Schwingungserreger (5) durch Druckluft antreibbar bzw. erregbar ist und die Steuervorrichtung (72) durch ein Steuerventil (65) gebildet ist, dessen Ventilschieber (66) die Größe einer Ventilöffnung (67) steuert, die in einer sich zum Schwingungserreger (5) erstreckenden Zuführungsleitung (15b) für Druckluft angeordnet ist.

6. Medizinisches oder dentalmedizinisches Behandlungsinstrument nach Anspruch 5,
**dadurch gekennzeichnet,**
**daß** das Steuerventil (65) einen Ventilschieber (66) aufweist, der vorzugsweise axial bewegbar gelagert und durch den am Schwingungserreger (5) anstehenden Druck (p1) gegen die Kraft einer Feder (65) beaufschlagt ist und eine Steuerkante (88) zum Steuern der Größe der Ventilöffnung (67) aufweist.

7. Medizinisches oder dentalmedizinisches Behandlungsinstimment nach den Ansprüchen 2 und 5 oder 6,
**dadurch gekennzeichnet,**
**daß** eim geneinsamer Ventilschieber (66) für dem Druckregler (71a) und die steuervorrichtung (72) vorgesehen ist.

8. Medizinisches oder dentalmedizinisches Behandlungsinstrument (1) nach einem der Ansprüche 3 bis 7,
**dadurch gekennzeichnet,**
**daß** die Steuervorrichtung (72) auf den Druckregler (71a) oder auf den Ventilschieber (66) einwirkt.

9. Medizinisches oder dentalmedizinisches Behandlungsinstrument (1) nach Anspruch 8,
**dadurch gekennzeichnet,**
**daß** die Kraft der Feder (68) durch die Steuervorrichtung (72) veränderlich ist.

10. Medizinisches oder dentalmedizinisches Behandlungsinstrument nach einem der vorherigen Ansprüche 4 bis 9,
**dadurch gekennzeichnet,**
**daß** die Einstellvorrichtung (73) und/oder das Steuerventil (65) und/oder das Druckregelventil (71b) im Handstück (2) angeordnet ist bzw. sind.

11. Medizinisches oder dentalmedizinisches Behandlungsinstrument nach Anspruch 10,
**dadurch gekennzeichnet,**
**daß** das Einstellglied (74) im hinteren Endbereich des Handstücks (2) angeordnet ist.

12. Medizinisches oder dentalmedizinisches Behandlungsinstrument nach einem der Ansprüche 4 bis 11,
**dadurch gekennzeichnet,**
**daß** das Einstellglied (74) an der Mantelfläche des Handstücks (2) angeordnet ist oder die Mantelfläche bildet und vorzugsweise in Umfangsrichtung verstellbar ist.

13. Medizinisches oder dentalmedizinisches Behandlungsinstrument nach Anspruch 12,
**dadurch gekennzeichnet,**
**daß** das Einstellglied (74) durch eine Hülse (74a) gebildet ist.

14. Medizinisches oder dentalmedizinisches Behandlungsinstrument nach einem der vorherigen Ansprüche 4 bis 17,
**dadurch gekennzeichnet,**
**daß** die Einstellvorrichtung (73) ein im Handstück (2) beweglich gelagertes Verstellglied (81) aufweist, das durch ein Untersetzungsgetriebe mit dem Einstellglied (74) verbunden ist.

15. Medizinisches oder dentalmedizinisches Behandlungsinstrument nach einem der vorherigen Ansprüche 4 bis 14,
**dadurch gekennzeichnet,**
**daß** die Einstellvorrichtung (73) ein im Handstück (2) beweglich, vorzugsweise axial beweglich, gelagertes Verstellglied (81) aufweist, das durch einen Mechanismus mit dem Einstellglied (74) verbunden ist, der die Bewegungsrichtung des Verstellgliedes 2 (81) bezüglich der des Einstellgliedes (74) verändert.

16. Medizinisches oder dentalmedizinisches Behandlungsinstrument nach einem der vorherigen An-sprüche 4 bis 15,
**dadurch gekennzeichnet,**
**daß** vom Einstellglied (74) ein Steg (76) radial nach innen durch einen Schlitz (77a) der Griffhülse (41) ragt, der eine Schräg- oder Kurvenfläche (79) aufweist, vorzugsweise an seiner Rückseite oder Vorderseite.

17. Medizinisches oder dentalmedizinisches Behandlungsinstrument nach einem der vorherigen Ansprüche 4 bis 16,
**dadurch gekennzeichnet,**
**daß** das Einstellglied (74a) mit einem Verstellglied (81) verbunden ist, das ein Widerlager für die Feder (68) des Steuerventils (65) bildet.

18. Medizinisches oder dentalmedizinisches Behandlungsinstrument nach einem der Ansprüche 4 bis 17,
**dadurch gekennzeichnet,**
**daß** der Einstellvorrichtung (73) eine manuell überdrückbare Feststellvorrichtung (83) oder Verrastungsvorrichtung (84) zum Sichern in der jeweils eingestellten Position zugeordnet ist.

19. Medizinisches oder dentalmedizinisches Behandlungsinstrument nach einem der vorherigen Ansprüche 4 bis 18,
**dadurch gekennzeichnet,**
**daß** auf dem Verstellweg des Einstellglieds (74) eine Markierung (155) und/oder Skalierung jeweils mit einer Gegenmarkierung oder mit einer Gegenskalierung bzw. einem Index und/oder eine oder mehrere Raststellen (166) am Einstellglied (74) und einem ihm benachbarten Teil, insbesondere dem das Einstellglied (74) tragenden Teil, angeordnet ist bzw. sind.

20. Medizinisches oder dentalmedizinisches Behandlungsinstrument nach Anspruch 19,
**dadurch gekennzeichnet,**
**daß** die Markierung (155) unterschiedliche Zeichen oder Farben aufweist.

21. Medizinisches oder dentalmedizinisches Behandlungsinstrument nach Anspruch 19 oder 20, in Kombination mit
mehreren unterschiedlichen Werkzeugen (7) die sich z.B. bezüglich ihrer Form und/oder Größe und/oder Festigkeit und/oder Abtragsfähigkeit voneinander unterscheiden.

22. Medizinisches oder dentalmedizinisches Behandlungsinstrument nach Anspruch 21,
**dadurch gekennzeichnet,**
**daß** die Werkzeuge (7) vorzugsweise jeweils am Werkzeugschaft (8) eine Markierung (156) aufweisen, wobei die Position der Markierung (155) und/oder der Raststellen (166) auf dem Verstellweg einem bestimmten spezifischen Unterscheidungsmerkmal des zugehörigen Werkzeugs (7) entspricht.

## Claims

1. Medical or dental-medical treatment instrument (1) for the material-removing working of body tissue or a substitute material, having an abrasive tool (7), consisting of a handpiece (2) in the forward end region of which there is mounted an oscillation part (4) carrying the tool (7), which can be set into oscillation by means of an oscillation generator (5) in the handpiece (5), the handpiece (2) being connected or connectable at its rearward end region with a flexible supply line (18) which extends from a supply device, and the oscillation generator (5) being driveable or excitable by means of compressed air which is delivered through a delivery line (15b) extending through the handpiece (2),
**characterized in that**,
there is provided in the delivery line (15b) a pressure regulator (71a) which even in the presence of differently high operating pressures (p2) regulates a substantially constant working pressure (p1) effective at the oscillation generator (5),
the pressure regulator (71a), and therewith the power of the oscillation generator (5), being adjustable.

2. Medical or dental-medical treatment instrument (1) according to claim 1,
**characterized in that**,
the pressure regulator (71a) includes a pressure regulation valve (71b) having a valve slider (66) which is mounted displaceably, preferably axially, by the working pressure (p1) against the force of a spring (68), and in the case of a greater operating pressure (p2) controls to a smaller valve opening (67) and in the case of a smaller operating pressure (p2) controls to a greater valve opening (67).

3. Medical or dental-medical treatment instrument (1) according to any preceding claim,
**characterized in that**,
there is provided a control device (72) with which the power can be increased or decreased.

4. Medical or dental-medical treatment instrument according to claim 3,
**characterized in that**,
there is provided a setting device (73) having a manually adjustable setting member (74) which acts upon the control device (72) or constitutes the control device (72).

5. Medical or dental-medical treatment instrument according to claim 3 or 4,
**characterized in that**,
the oscillation generator (5) is driveable or excitable by means of compressed air and the control device (72) is constituted by means of a control valve (65) the valve slider (66) of which controls the size of a valve opening (67) which is arranged in a delivery line (15b) for compressed air extending to the oscillation generator (5).

6. Medical or dental-medical treatment instrument according to claim 5,
**characterized in that**,
the control valve (65) has a valve slider (66) which is preferably axially movably mounted and is acted upon by the pressure (p1) effective at the oscillation generator (5) against the force of a spring (65) and has a control edge (88) for controlling the size of the valve opening (67).

7. Medical or dental-medical treatment instrument according to claims 2 and 5 or 6,
**characterized in that**,
there is provided a common valve slider (66) for the pressure regulator (71a) and the control device (72).

8. Medical or dental-medical treatment instrument (1) according to any of claims 3 to 7, **characterized in that**,
the control device (72) acts upon the pressure regulator (71a) or upon the valve slider (66).

9. Medical or dental-medical treatment instrument (1) according to claim 8,
**characterized in that**,
the force of the spring (68) can be varied by the means of the control device (72).

10. Medical or dental-medical treatment instrument according to any of preceding claims 4 to 9,
**characterized in that**,
the setting device (73) and/or the control valve (65) and/or the pressure regulating valve (71b) is or are arranged in the handpiece (2).

11. Medical or dental-medical treatment instrument according to claim 10,
**characterized in that**,
the setting member (74) is arranged in the rearward end region of the handpiece (2).

12. Medical or dental-medical treatment instrument according to any of claims 4 to 11,
**characterized in that**,
the setting member (74) is arranged on the outer surface of the handpiece (2), or forms the outer surface, and is preferably adjustable in the circumferential direction.

13. Medical or dental-medical treatment instrument according to claim 12,
**characterized in that**,
the setting member (74) is formed by means of a sleeve (74a).

14. Medical or dental-medical treatment instrument according to any of preceding claims 4 to 13,
**characterized in that**,
the setting device (73) has an adjustment member (81) movably mounted in the handpiece (2), which adjustment member is connected with the setting member (74) by means of a step-down transmission.

15. Medical or dental-medical treatment instrument according to any of preceding claims 4 to 14,
**characterized in that**,
the setting device (73) has an adjustment member (81) mounted movably, preferably axially, in the handpiece (2), which adjustment member is connected with the setting member (74) by means of a mechanism which alters the direction of movement of the adjustment member (81) with regard to that of the setting member (74).

16. Medical or dental-medical treatment instrument according to any of preceding claims 5 to 16,
**characterized in that**,
from the setting member (74) a web (76) projects radially inwardly through a slot (77a) of the grip sleeve (41), which has an oblique or curved surface (79), preferably at the rearward side or forward side.

17. Medical or dental-medical treatment instrument according to any of preceding claims 4 to 16,
**characterized in that**,
the setting member (74a) is connected with an adjustment member (81) which forms an abutment for the spring (68) of the control valve (65).

18. Medical or dental-medical treatment instrument according to any of claims 4 to 17,
**characterized in that**,
there is associated with the setting device (73) a fixing device (83) or a latching device (84), which can be manually overcome, for securing in the respectively set position.

19. Medical or dental-medical treatment instrument according to any of preceding claims 4 to 18,
**characterized in that**,
there is or are arranged on the adjustment path of the setting member (74) a marking (155) and/or a scale in each case with a counter-marking or a counter-scale or an index and/or one or more latching points (166) on the setting member (74) and a part neighbouring thereto, in particular the part carrying the setting member (74).

20. Medical or dental-medical treatment instrument according to claim 19,
**characterized in that**,
the marking (155) has different characters, or colours.

21. Medical or dental-medical treatment instrument according to claim 19 or 20,
in combination with,
a plurality of different tools (7) which differ from one another for example with regard to their shape and/or size and/or strength and/or material-removal capabilities.

22. Medical or dental-medical treatment instrument according to claim 21,
**characterized in that**,
the tools (7) have, preferably in each case on the tool shaft, (8) a marking (156), the positioning of the marking (155) and/or of the latch points (166) on the adjustment path corresponding to a certain specific distinguishing feature of the associated tool (7).

## Revendications

1. Instrument de traitement médical ou dentaire par enlèvement (1) de tissus corporels ou d'un matériau de remplacement doté d'un outil d'abrasion (7), constitué d'un porte-outil (2) à l'extrémité antérieure duquel est logé un élément oscillant qui porte l'outil (7) et que l'on peut faire osciller grâce à un oscillateur (5) dans lequel le porte-outil (2) est connecté ou connectable, au niveau de son extrémité postérieure, à une ligne d'alimentation flexible (18) sortant d'un dispositif d'alimentation, en sorte que l'oscillateur (5) peut être activé, respectivement commandé par air comprimé qui peut être introduit par une conduite d'amenée (15b), s'étendant à travers le porte-outil (2),
**caractérisé en ce que**,
dans la conduite d'amenée (15b) est prévu un régulateur de pression (71a) assurant qu'une pression de travail (p1) sensiblement constante est appliquée à l'oscillateur (5) même en présence de pressions de fonctionnement (p2) distinctement plus élevée, et
**en ce que** le régulateur de pression (71a) et de fait la puissance de l'oscillateur (5) est réglable.

2. Instrument de traitement médical ou dentaire (1) selon la revendication 1,
**caractérisé en ce que**
le régulateur de pression (71a) comporte une soupape de régulation de pression (71b) dotée d'une vanne de soupape (66) logée de façon à pouvoir être déplacée par la pression de travail (p1), de préférence, dans le sens axial, à l'encontre de la force d'un ressort (68) et qui, en présence d'une pression de fonctionnement supérieure, commande une ouverture de soupape (67) inférieure et, en présence d'une pression de régime inférieure, commande une ouverture de soupape (67) supérieure.

3. Instrument de traitement médical ou dentaire (1) selon l'une quelconque des revendications précédentes,
**caractérisé en ce qu'**il est prévu un dispositif de commande (72) au moyen duquel la puissance peut être augmentée ou diminuée.

4. Instrument de traitement médical ou dentaire selon la revendication 3,
**caractérisé en ce qu'**il est prévu un dispositif de réglage (73), doté d'un élément de réglage (74) ajustable manuellement, qui agit sur le dispositif de commande (72) ou qui constitue le dispositif de commande (72).

5. Instrument de traitement médical ou dentaire la revendication 3 ou la revendication 4,
**caractérisé en ce que**
l'oscillateur (5) peut être activé, respectivement, commandé par air comprimé et **en ce que** le dispositif de commande (72) est constitué par une soupape de distribution (65) dont la vanne de soupape (66) commande la taille d'une ouverture de soupape (67) qui est agencée dans une conduite d'amenée (15b) d'air comprimé allant jusqu'à l'oscillateur (5).

6. Instrument de traitement médical ou dentaire selon la revendication 5,
**caractérisé en ce que**
la soupape de distribution (65) présente une vanne de soupape (66) qui est logée de façon à pouvoir se déplacer, de préférence, dans le sens axial, qui est alimentée contre la force d'un ressort (65), par la pression apparaissant au niveau de l'oscillateur (5) et qui présente une arrête de commande (88) servant à contrôler la taille de l'ouverture de soupape (67).

7. Instrument de traitement médical ou dentaire selon les revendications 2 et 5 ou 6,
**caractérisé en ce qu'**il est prévu une vanne de soupape (66) commune pour le régulateur de pression (71a) et le dispositif de commande (72).

8. Instrument de traitement médical ou dentaire (1) selon l'une quelconque des revendications 3 à 7,
**caractérisé en ce que**
le dispositif de commande (72) agit sur le régulateur de pression (71a) ou sur la vanne de soupape (66).

9. Instrument de traitement médical ou dentaire (1) selon la revendication 8,
**caractérisé en ce que**
l'on peut faire varier la force du ressort (68) au moyen du dispositif de commande (72).

10. Instrument de traitement médical ou dentaire selon l'une quelconque des revendications 4 à 9,
**caractérisé en ce que**
le dispositif de réglage (73) et/ou la soupape de distribution (65) et/ou la soupape de régulation de pression (71b) est, respectivement, sont agencé(s) dans le porte-outil (2).

11. Instrument de traitement médical ou dentaire selon la revendication 10,
**caractérisé en ce que**
l'élément de réglage (74) est placé à l'extrémité postérieure du porte-outil (2).

12. Instrument de traitement médical ou dentaire selon l'une quelconque des revendications 4 à 11,
**caractérisé en ce que**
l'élément de réglage (74) est disposé au niveau de la surface latérale du porte-outil (2) ou constitue cette surface latérale et **en ce qu'**il est déplaçable, de préférence, dans la direction circonférentielle.

13. Instrument de traitement médical ou dentaire selon la revendication 12,
**caractérisé en ce que**
l'élément de réglage (74) est constitué par un manchon (74a).

14. Instrument de traitement médical ou dentaire selon l'une quelconque des revendications 4 à 11,
**caractérisé en ce que**
le dispositif de réglage (73) présente un élément d'ajustement (81) logé de façon mobile dans le porte-outil (2), cet élément d'ajustement étant relié à l'élément de réglage (74) par un organe d'appui.

15. Instrument de traitement médical ou dentaire selon l'une quelconque des revendications 4 à 14,
**caractérisé en ce que**
le dispositif de réglage (73) présente un élément d'ajustement (81) logé de façon mobile, de préférence de façon axialement mobile, dans le porte-outil (2), cet élément d'ajustement étant relié à l'élément de réglage (74) par un mécanisme qui modifie la direction de déplacement de l'élément d'ajustement (81) par rapport à celui de l'élément de réglage (74).

16. Instrument de traitement médical ou dentaire selon l'une quelconque des revendications 4 à 15,
**caractérisé en ce qu'**un pont (76) s'étend radialement à partir de l'élément de réglage (74), vers l'intérieur par une fente (77a) du manchon de prise, ce pont présentant un galbe ou une courbure (79), de préférence au niveau de sa face arrière ou de sa face avant.

17. Instrument de traitement médical ou dentaire selon l'une quelconque des revendications 4 à 16,
**caractérisé en ce que**
l'élément de réglage (74a) est relié à un élément d'ajustement (81) qui sert de butée pour le ressort (68) de la soupape de distribution (65).

18. Instrument de traitement médical ou dentaire selon l'une quelconque des revendications 4 à 17,
**caractérisé en ce que**
le dispositif de réglage (73) est complété par un dispositif de verrouillage (83) ou un dispositif d'enclenchement (84) pouvant être repoussé manuellement servant au blocage dans l'une ou l'autre des positions ayant été ajustées.

19. Instrument de traitement médical ou dentaire selon l'une quelconque des revendications 4 à 18,
**caractérisé en ce que**
sur la course de réglage de l'élément de réglage (74), est, respectivement, sont disposé(s) un marquage (155) et/ou une graduation, respectivement accompagnés d'un contre-marquage ou d'une contre-graduation, respectivement d'un index, et/ou une ou plusieurs positions d'enclenchement (166), au niveau de l'élément de réglage (74) et d'une pièce voisine de celui-ci, particulièrement au niveau de la pièce portant l'élément de réglage (74).

20. Instrument de traitement médical ou dentaire selon la revendication 19,
**caractérisé en ce que**
le marquage (155) présente différents signes ou couleurs.

21. Instrument de traitement médical ou dentaire selon la revendication 19 ou 20 combiné à plusieurs outils différents (7) qui se distinguent les uns des autres, par exemple, du point de vue de leur forme et/ou de leur taille et/ou de leur résistance et/ou de leur capacité d'abrasion.

22. Instrument de traitement médical ou dentaire selon la revendication 21,
**caractérisé en ce que**
les outils (7) présentent, de préférence, respectivement au niveau de la tige d'outil (8), une marque (156), en sorte que la disposition du marquage (155) et/ou des positions d'enclenchement (166) sur la course de réglage correspond à un certain caractère distinctif spécifique de l'outil (7) correspondant.
